# EUROPÄISCHE PATENTSCHRIFT

(11) **EP 3 552 011 B1**
(45) Veröffentlichungstag und Bekanntmachung des Hinweises auf die Patenterteilung: **30.12.2020**
(21) Anmeldenummer: 17801701.8
(22) Anmeldetag: 20.11.2017
(51) Int. Cl.: G01N 33/00, G01M 15/02

(54) **ANALYSEEINHEIT**
ANALYSIS UNIT
UNITÉ D'ANALYSE

(30) Priorität: 09.12.2016 DE 102016123925
(43) Veröffentlichungstag der Anmeldung: 16.10.2019
(73) Patentinhaber: AVL Emission Test Systems GmbH, 41460 Neuss (DE)
(72) Erfinder: MARTINEWSKI, Georg, 40217 Düsseldorf (DE)
(74) Vertreter: terpatent Patentanwälte ter Smitten Eberlein-Van Hoof Rütten Daubert
(86) Internationale Anmeldenummer: PCT/EP2017/079759
(87) Internationale Veröffentlichungsnummer: WO 2018/104037

(56) Entgegenhaltungen:
- DE-U1-202009 014 666
- "ACX Analyzer Systems for Emission Monitoring, Cement Applications and Process Measurement", , 1. Mai 2007 (2007-05-01), XP055056544, Gefunden im Internet: URL:http://www.cemsi.on.ca/Files/ACX_data. pdf [gefunden am 2013-03-14]
- Siemens ET AL: "Siemens PA 01 . 2015 Analytical Application Sets", , 1. Januar 2013 (2013-01-01), XP055245324, Gefunden im Internet: URL:http://www.automation.siemens.com/sc-s tatic/catalogs/catalog/pa/PA01/en/PA01_en_ Kap04.pdf [gefunden am 2016-01-27]

## Beschreibung

Die Erfindung betrifft eine Analyseeinheit mit einer Frontalebene, von der aus die Analyseeinheit zugänglich ist und parallelen Befestigungsebenen, welche senkrecht zur Frontalebene angeordnet sind und einem Gasmessgerät.

Derartige Analyseeinheiten sind üblicherweise in Schaltschränken angeordnet, welche an ihrer Frontseite eine Tür aufweisen, über die der Monteur oder das Bedienpersonal Zugang zu den einzelnen Messgeräten und deren Peripherieelementen erhält, so dass diese möglichst von der Frontseite erreichbar angebracht sind. Die Messgeräte und Peripherieelemente setzen sich zumeist aus mehreren Gehäuseteilen zusammen, von denen zumindest eines beispielsweise an einem der Schrankböden, welches dann als Befestigungsebene dient, befestigt wird, während die weiteren Gehäuseteile an dieses Teil montiert werden müssen. Je nach vorhandenem Bauraum ist dabei gegebenenfalls die Zugänglichkeit zu einzelnen Geräten nur eingeschränkt möglich, so dass sich insbesondere bei der Verwendung von Werkzeugen die Montage und Demontage schwierig gestaltet.

So weist beispielsweise ein Flammenionisationsdetektor, wie er beispielsweise in der DE 196 21 293 A1 beschrieben wird, neben dem Raum, in dem die Flamme oder die Flammen erzeugt werden und den dorthin führenden Gas- und Stromleitungen zur Versorgung der Elektroden sowie der Elemente zur Bestimmung der bei der Ionisierung freiwerdender Elektronen und der angeschlossenen elektronischen Auswerteeinheit einen Konverter auf, in welchem Kohlenwasserstoffmoleküle mit Ausnahme vor Methan mit Wasserstoffmolekülen und Sauerstoffmolekülen an einer Katalysatorfläche in Kohlendioxid und Wasser umgewandelt werden. Zur Bestimmung der Kohlenwasserstoffe wird eine Flamme des Flammenionisationsdetektors vom Abgasstrom durchströmt, während die andere Flamme von dem durch den Konverter geführten Abgasstrom durchströmt wird, so dass lediglich die Methananteile im Abgas an der anderen Flamme bestimmt werden, wobei die Umsetzungsgrade des Konverters zu beachten sind, welche bezüglich des Methans bei etwa 13 % liegen. Durch Differenzbildung kann daraufhin die Menge der Kohlenwasserstoffe ohne den Methananteil bestimmt werden. Obwohl diese Bauteile zu einem Flammenionisationsdetektor zusammengebaut werden, weisen sie verschiedene Gehäuseteilungen auf, um eine korrekte Montage sicherstellen zu können und bei Funktionsstörungen auch eine Zugänglichkeit einzelner Elemente des Flammenionisationsdetektors, wie die Katalysatoren des Konverters, sicherstellen zu können, ohne diesen komplett ausbauen zu müssen.

Des Weiteren ist aus der Veröffentlichung "Advanced CGA Solutions ACX Analyzer Systems for Emission Monitoring, Cement Applications and Process Measurement" eine Analyseeinheit bekannt, welche ein Gasmessgerät in einem Schrank aufweist, welches eine Befestigungsebene aufweist, über die die Gasanschlüsse erfolgen.

Aus der Veröffentlichung "Siemens PA 01 2015 Analytical Application Sets" ist ebenfalls eine in einem Schaltschrank angeordnete Analyseeinheit bekannt, deren Gasmessgerät in nicht näher dargestellter Weise mit dem Probengasstrom versorgt wird.

Auch die DE 20 2009 014 666 U1 offenbart einen Schaltschrank zur Abgasmessung, in dem entsprechend eine Analyseeinheit und ein Gasmessgerät angeordnet sind.

Nachteilig an den bekannten Systemen ist es, dass der Einbau de Messgeräte beziehungsweise der Gehäuseteile der Geräte unabhängig vom zur Verfügung stehenden Bauraum immer festliegt. Zusätzlich besteht häufig eine sehr schlechte Zugänglichkeit, wodurch Montagezeiten und Wartungszeiten deutlich erhöht werden.

Es stellt sich daher die Aufgabe, eine Analyseeinheit so auszugestalten,dass die Messgeräte und/oder deren Peripheriegeräte einfach ein- und ausbaubar sind, indem die Zugänglichkeit dieser Geräte verbessert wird und dass es gleichzeitig möglich wird, die Einbaurichtung an den vorhandenen Bauraum anzupassen, ohne hierfür die Gehäuseteile ändern zu müssen.

Diese Aufgabe wird durch eine Analyseeinheit mit den Merkmalen: des Hauptanspruchs 1 gelöst.

Dadurch, dass das Gasmessgerät zumindest ein Anschlussgehäuseteil aufweist, durch welches sich ein Einlasskanal von der Befestigungsebene zu einer Anschlussebene und ein Auslasskanal von der Anschlussebene bis zur Befestigungsebene erstreckt, und ein Grundgehäuseteil mit einer Anschlussebene aufweist, die der Anschlussebene des Anschlussgehäuseteils entspricht und an der das Grundgehäuseteil gegen das Anschlussgehäuseteil anliegt, wobei im Grundgehäuseteil ein Zuströmkanal ausgebildet ist, der fluidisch über die Anschlussebene mit dem Einlasskanal des Anschlussgehäuseteils verbunden ist und ein Rückströmkanal ausgebildet ist, der fluidisch über die Anschlussebene mit dem Auslasskanal des Anschlussgehäuseteils verbunden ist, wobei die korrespondierenden Anschlussebenen im 45°-Winkel von der Befestigungsebene in Richtung zur Frontalebene gekippt angeordnet sind, bleibt ein Flansch, über den die beiden Gehäuseteile miteinander verbunden werden, von der Frontseite aus gut zugänglich. Insbesondere bleibt im Frontbereich Platz zur Nutzung von Werkzeugen, wie Schraubenschlüssel. Zusätzlich kann bei korrekter Anordnung der Gas führenden Kanäle das Grundgehäuse in zwei Richtungen auf das Anschtussgehäuseteil aufgebracht und befestigt werden, indem das Grundgehäuseteil um die eigene Achse gedreht und um 90° gekippt wird. So können zwei im 90°-Winkel zueinander stehende Erstreckungsrichtungen des Grundgehäuseteils bei der Montage hergestellt werden, wodurch eine Befestigung in Abhängigkeit des vorhandenen Bauraums ermöglicht wird.

Vorzugsweise sind alle Bohrungen, Kanäle, Aufnahmeöffnungen oder Ausnehmungen im Anschlussgehäuseteil und im Grundgehäuseteil symmetrisch zu einer Symmetrieebene angeordnet, die senkrecht zur Befestigungsebene verläuft. Auf diese Weise wird für beide alternativen Einbaurichtungen eine korrekte Gasführung sichergestellt, ohne die aneinander zu befestigenden Bauteile anpassen zu müssen.

Des Weiteren ist es vorteilhaft, wenn das Grundgehäuseteil eine Flanschplatte aufweist, deren erste Flanschfläche die Anschlussebene bildet und deren zweite Flanschfläche parallel zur Anschlussebene verläuft, wobei sich Bohrungen von der ersten Flanschfläche zur zweiten Flanschfläche durch die Flanschplatte erstrecken. Unabhängig von der Anbaurichtung des Grundgehäuseteils sind die Einsteckrichtung der Schrauben und damit die Richtung der Kraft zur Befestigung durch die Schrauben immer im 45°-Winkel zur Erstreckungsrichtung des Gehäuseteils ausgerichtet. Somit sind die Flanschfläche und damit die Schraubenköpfe zur Befestigung immer gut von der Frontseite aus zugänglich.

Um die Montage besonders einfach durch ein Eindrehen der Schrauben sicherstellen zu können, sind in einer weiterführenden Ausführungsform am Anschlussgehäuseteil zwei Gewindebohrungen ausgebildet, in welche Schrauben drehbar sind, die sich durch die Bohrungen der Flanschplatte in die Gewindebohrungen erstrecken.

Des Weiteren ist es vorteilhaft, wenn sich ein Körper des Grundgehäuseteils zwischen den beiden Bohrungen im 45° Winkel zu den Flanschflächen erstreckt, wobei das Grundgehäuseteil in zwei senkrecht zueinander angeordneten Positionen am Anschlussgehäuseteil befestigbar ist. Somit erstreckt sich das Grundgehäuseteil entweder im Schrank senkrecht zur Befestigungsebene beziehungsweise zum Schrankboden oder zur Frontseite. Je nach vorhandenem Bauraum beziehungsweise verwendetem Schaltschrank kann auf diese Weise mit Gleichteilen eine Montage erfolgen, wodurch Produktionskosten gesenkt werden können.

In einer weiterführenden Ausbildung der Erfindung geht die schräge Anschlussebene des Anschlussgehäuseteils in eine Auflagefläche über, die parallel zur Befestigungsebene angeordnet sind. Entsprechend geht die schräge Anschlussebene des Grundgehäuseteils in eine Fläche über, die um 45° zur Anschlussebene abgewinkelt ist und die auf die Auflagefläche des Anschlussgehäuseteils in einer ersten Befestigungsposition aufsetzbar ist. Dies erleichtert den Montagevorgang, da das Grundgehäuseteil auf das Anschlussgehäuseteil bei der Montage aufgesetzt werden kann, ohne festgehalten werden zu müssen. Dennoch bleibt die schräge Anschlussebene zur Verbesserung der Erreichbarkeit.

In einer bevorzugten Ausbildung der Erfindung ist im Zuströmkanal und/oder im Rückströmkanal des Grundgehauseteils Konvertermaterial angeordnet, welches zur Umwandlung von Kohlenwasserstoffen in Methan dient. Entsprechend dient das Grundgehäuseteil als Gehäuse eines Konverters, der entsprechend einfach montiert werden kann und dessen Erreichbarkeit beispielswiese zum Austausch des Konvertermaterials erhalten bleibt, ohne hierfür den gesamten Flammenionisationsdetektor ausbauen zu müssen. Durch die Anordnung des Konvertermaterials in zwei parallelen Kanälen kann die Länge eines Zylinders gekürzt und damit das benötigte Volumen zur Umwandlung auf kleinem Bauraum zur Verfügung gestellt werden.

Diese Minimierung, insbesondere der axialen Baulänge eines Konverters, kann dadurch noch weiter gekürzt werden, dass im Grundgehäuseteil zwischen dem Zuströmkanal und dem Rückströmkanal zwei weitere parallel verlaufende Durchströmungskanäle ausgebildet sind, in denen Konvertermaterial angeordnet ist und die mit dem jeweils benachbarten Zuströmkanal beziehungsweise Rückströmkanal über axiale Ausnehmungen zwischen den vom Anschlussgehäuseteil weg weisenden Enden des Zuströmkanals und des benachbarten Durchströmungskanals und des Rückströmkanals und des benachbarten Durchströmungskanals miteinander verbunden sind.

Eine vollständige Durchströmung aller vier Gaskanäle mit Konvertermaterial wird sichergestellt, indem im Grundgehäuseteil am zum Anschlussgehäuseteil weisenden Ende ein Verbindungskanal zwischen den beiden Durchströmungskanälen ausgebildet ist, so dass das Grundgehäuseteil mäanderförmig durchströmt ist. Entsprechend werden die Konverterkanäle nacheinander durchströmt, ohne das Grundgehäuseteil zu verlassen.

Vorzugsweise ist an der Anschlussebene des Anschlussgehäuseteils eine erste kreisförmige Ausnehmung ausgebildet, in die der Einlasskanal mündet und welche mit dem Zuströmkanal des Grundgehäuseteils fluidisch verbunden ist und eine zweite kreisförmige Ausnehmung ausgebildet, in die der Rückströmkanal des Grundgehäuseteils mündet und welche mit dem Auslasskanal des Anschlussgehäuseteils fluidisch verbunden ist, und eine langlochförmige Ausnehmung ausgebildet, in die die beiden Durchströmungskanäle des Grundgehäuseteils münden. So wird der Gasstrom vor Erreichen des Konvertermaterials in den Kanälen des Grundgehäuseteils auf die gesamte Querschnittsfläche der das Konvertermaterial enthaltenden Kanäle erweitert, so dass hierin eine optimale Strömung erzielt wird. Zusätzlich wird ein erhöhter Druckverlust durch gleichmäßige zur Verfügung stehende Querschnitte vermieden.

In einer weiteren Ausbildung der Erfindung sind der Zuströmkanal, der Rückströmkanal und die beiden Durchströmungskanäle mittels einer an ihrem zum Anschlussgehäuseteil entgegengesetzten Ende aufgeschraubten Platte verschlossen. Diese erleichtert die Herstellung des Grundgehäuseteils, in welches die Kanäle durch einfaches Bohren und Fräsen eingebracht werden können und somit die Zugänglichkeit und damit die Füllung der Kanäle mit dem Konvertermaterial.

Des Weiteren kann vorteilhafterweise im Grundgehäuseteil zumindest eine erste Aufnahmeöffnung ausgebildet sein, in der eine Heizpatrone angeordnet ist und zumindest eine zweite Aufnahmeöffnung ausgebildet sein, in der ein Temperaturfühler angeordnet ist. Auf diese Weise kann im Konverter eine bevorzugte Temperatur von etwa 280°C erzeugt und überprüft werden, um eine optimale Umsetzung der Kohlenwasserstoffe sicherstellen zu können.

Vorzugsweise ist das Gasmessgerät ein Flammenionisationsdetektor und das Grundgehäuseteil bildet entsprechend einen Konverter. Dieses Gasmessgerät ist üblicherweise aus verschiedenen Gehäuseteilen aufgebaut, deren Zugänglichkeit möglichst einzeln sicherzustellen ist, um eine Wartung vornehmen zu können.

Es wird somit eine Analyseeinheit geschaffen, die aufgrund deutlich verbesserter zugänglichkeit sehr wartungs- und montagefreundlich ist. Zusätzlich werden aufgrund der möglichen zwei Anbaurichtungen Produktionsvorteile erzielt, da auch bei unterschiedlichen zur Verfügung stehenden Bauräumen optimale Einbaurichtungen mit Gleichteilen verwirklicht werden können. Besondere Vorteile werden insbesondere bei der Herstellung, Montage und Wartung eines Konverters eines Flammenionisationsdetektors erzielt.

Ein Ausführungsbeispiel einer erfindungsgemäßen Analyseeinheit ist in den Figuren schematisch dargestellt und wird nachfolgend beschrieben.

Die Figur 1 zeigt einen Ausschnitt eines Schaltschranks mit einer erfindungsgemäßen Analyseeinheit.

Die Figur 2 zeigt in perspektivischer Darstellung ein Anschlussgehäuseteil der erfindungsgemäßen Analyseeinheit aus Figur 1.

Die Figur 3 zeigt in perspektivischer Darstellung ein Grundgehäuseteil der erfindungsgemäßen Analyseeinheit aus Figur 1.

Die Figur 4 zeigt eine Draufsicht auf das Grundgehäuseteil der erfindungsgemäßen Analyseeinheit aus Figur 1 von unten.

Die dargestellte Analyseeinheit ist in einem Schaltschrank 10 angeordnet, der verschiedene Gasmessgeräte 12, 14, 16 enthält. Das obere dieser Gasmessgeräte 12 ist ein Flammenionisationsdetektor, in dessen Außengehäuse 18 mehrere weitere zu montierende Gehäuseteile 20, 22, 24 angeordnet sind. Mehrere zusammengesetzte Gehäuseteile 20 umgeben den Raum, in dem zwei Flammen des Flammenionisationsdetektors 12 ausgebildet sind, die notwendigen Gaszuleitungen und -ableitungen und die notwendige Elektronik zur Messung und Auswertung am Detektor. Über die beiden Flammen wird in bekannter Weise ein Anteil an Methan und ein Anteil der insgesamt vorhandenen Kohlenwasserstoffe bestimmt, indem das Messgas mit einem unter anderem Wasserstoff enthaltenden Trägergasstrom zur Flamme gefördert wird, wo eine thermische Ionisierung stattfindet, durch welche eine messbare Leitfähigkeit zwischen zwei Elektroden erzeugt wird, die ein Maß für die im Messgas vorhandenen Kohlenwasserstoffe ist.

Der Messgasstrom wird jedoch in einen ersten Teilstrom, der direkt zur ersten Flamme gefördert wird, so dass dort ein Gesamtstrom aller vorhandenen Kohlenwasserstoffe ermittelt werden kann und einen zweiten Teilstrom aufgeteilt, der über einen Konverter 26 einer zweiten Flamme zugeführt wird. Im Konverter 26 werden alle Kohlenwasserstoffe außer Methan an einem Konvertermaterial, welches beispielsweise Hopkalit sein kann, welches eine Mischung aus Kupferoxid und Manganoxid ist, bei Vorhandensein von Wasserstoff und Sauerstoff zu Kohlendioxid und Wasser umgewandelt. Während die übrigen Kohlenwasserstoffe beinahe vollständig umgewandelt werden, wird in diesem Konverter 26 das Methan lediglich zu etwa 13 % umgesetzt, so dass durch entsprechende Umrechnung der ermittelten Leitfähigkeit des Gases in der zweiten Flamme auf den Anteil des Methans an den gesamt vorhandenen Kohlenwasserstoffen geschossen werden kann.

Dieser Konverter 26 beinhaltet jedoch das Konvertermaterial, welches in entsprechend geformte Kanäle des Konverters 26 einzubringen ist. Zusätzlich ist dieses Material gegebenenfalls bei der Wartung der Messgeräte auszutauschen, so dass eine gute Zugänglichkeit gewünscht ist.

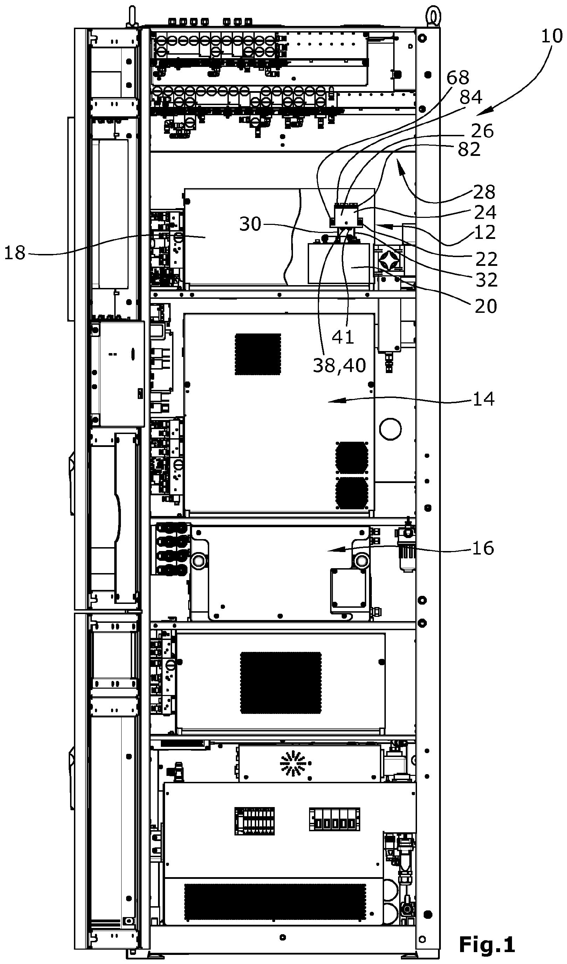

Wie aus der Figur 1 hervorgeht, kann von einer zugänglichen Frontalebene 28, die durch die Frontseite des Schaltschrankes 10 aufgespannt wird, Bedienpersonal das Innere des Schaltschrankes 10 erreichen. Von den Gehäuseteilen 20 erstrecken sich zwei Gasleitungen 30, 32 zu einem Anschlussgehäuse 22, in welchem ein Einlasskanal 34 und ein Auslasskanal 36 ausgebildet sind, in die jeweils eine der Gasleitungen 30, 32 münden. Dieses Anschlussgehäuse 22 ist auf einer als Befestigungsebene 38 dienenden Montageplatte 40 mittels Schrauben 41 befestigt, welche sich senkrecht zu einer Rückwand 42 des Schaltschrankes 10 erstreckt und entsprechend senkrecht zur zugänglichen Frontseite 28 des Schaltschrankes 10 angeordnet ist.

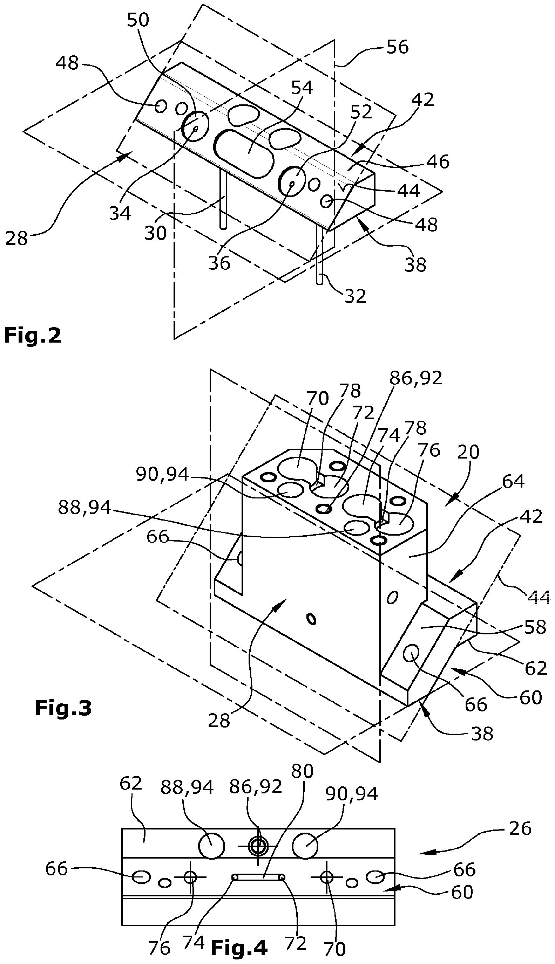

Das in der Figur 2 dargestellte Anschlussgehäuseteil 22 weist eine zur Befestigungsebene 38 um 45° in Richtung der Frontseite 28 gekippte Anschlussebene 44 auf, welche im zur Rückwand 42 weisenden Bereich in eine Auflagefläche 46 übergeht, die sich parallel zur Befestigungsebene 38 erstreckt. An der Anschlussebene 44 des Anschlussgehäuseteils 22 sind zwei Gewindebohrungen 48 im äußeren Bereich sowie eine erste kreisförmige Ausnehmung 50 ausgebildet, in die der Einlasskanal 34 mündet, eine zweite kreisförmige Ausnehmung 52 ausgebildet, von der aus sich der Auslasskanal 36 durch das Anschlussgehäuseteil 22 erstreckt und eine dazwischen angeordnete langlochförmige Ausnehmung 54 ausgebildet. Das Anschlussgehäuseteil 22 sowie die darin ausgebildeten Kanäle 34, 36, Bohrungen 48 und Ausnehmungen 50, 52, 54 sind jeweils symmetrisch zu einer Symmetrieebene 56 angeordnet, welche senkrecht zur Befestigungsebene 38 und zur Anschlussebene 44 verläuft und das Anschlussgehäuseteil 22 zentral in zwei gleich große Abschnitte teilt.

Auf dieses Anschlussgehäuseteil 22 wird ein Grundgehäuseteil 24 des Konverters 26 montiert, welches in den Figuren 3 und 4 dargestellt ist. Dieses weist eine Flanschplatte 58 auf, deren zum Anschlussgehäuseteil 22 weisende Flanschfläche 60 nach der Montage eine Anschlussebene aufweist, die der Anschlussebene 44 des Anschlussgehäuseteils 22 entspricht. Diese Flanschfläche 60 geht in eine Fläche 62 über, die wiederum auf der Auflagefläche 46 des Anschlussgehäuseteils 22 aufliegt und sich entsprechend ebenfalls parallel zur Befestigungsebene 38 erstreckt, so dass bei der Montage das Grundgehäuseteil 24 auf das Anschlussgehäuseteil 22 gestellt werden kann, denn ein Körper 64 des Konverters 26 erstreckt sich von der Flanschplatte 58 im 45°-Winkel nach oben, also senkrecht zu Befestigungsebene 38. In der Flanschplatte 58 ist rechts und links des Körpers 64 jeweils eine Bohrung 66 ausgebildet, die sich senkrecht zur Flanschfläche 60 durch die Flanschplatte 58 bis zu einer gegenüberliegenden Flanschfläche 64 erstreckt und durch welche bei der Montage des Grundgehäuseteils 24 an das Anschlussgehäuseteil 22 Schrauben 68 gesteckt und in die Gewindebohrungen 48 gedreht werden. Es ist ersichtlich, dass diese Schrauben 68 nicht nur gut von der Frontalebene 28 aus erreichbar sind, sondern auch ausreichend Platz für die Drehung eines Schraubenschlüssels durch die schräge Anordnung vorhanden ist.

Das Grundgehäuseteil 24 weist vier parallel zueinander verlaufende Kanäle 70, 72, 74, 76 auf, wovon ein erster einen Zuströmkanal 70 bildet, der über die axiale Ausnehmung 50 mit dem Einlasskanal 34 des Anschlussgehäuseteils 22 fluidisch verbunden ist, die beiden mittleren Kanäle als Durchströmungskanäle 72, 74, dienen und der andere äußere Kanal als Rückströmkanal 76 dient, der wiederum über die axiale kreisförmige Ausnehmung 52 mit dem Auslasskanal 36 fluidisch verbunden ist. Der Zuströmkanal 70 ist mit dem benachbarten Durchströmungskanal 72 ebenso wie der Rückströmkanal 76 mit dem benachbarten Durchströmungskanal 74 über axiale Ausnehmungen 78 miteinander verbunden, welche an der Trennwand zwischen den jeweils beiden Kanälen 70, 72, 74, 76 am vom Anschlussgehäuseteil 22 weg weisenden Ende des Körpers 64 ausgebildet sind. Am entgegengesetzten, also zum Anschlussgehäuse 22 weisenden Ende des Körpers 64 ist ein Verbindungskanal 80 zwischen den beiden Durchströmungskanälen 72, 74 ausgebildet, der gegenüberliegend zu der langlochförmigen Ausnehmung 54 des Anschlussgehäuseteils angeordnet ist. Das axiale Ende des Körpers 64 wird durch eine Platte 82 verschlossen, welche mittels Schrauben 84 auf dem Ende des Körpers 64 des Grundgehäuseteils 24 befestigt wird und so die Kanäle 70, 72, 74, 76 verschließt. Dies hat zur Folge, dass bei Eintritt eines Gases in den Einlasskanal 34 des Anschlussgehäuseteils 22 dieses in den Zuströmkanal 70 strömt um anschließend mäanderförmig durch die beiden Durchströmungskanäle 72, 74 in den Rückströmkanal 76 und von dort in den Auslasskanal 36 zu strömen. In allen diesen vier Kanälen 70, 72, 74, 76 ist jeweils das bereits oben beschriebene Konvertermaterial zur Beschleunigung der Reaktion vorhanden. Auf diese Weise werden eine ausreichende Reaktionsstrecke und ausreichend viel Konvertermaterial für eine vollständige Umsetzung der Nichtmethan-Kohlenwasserstoffe zur Verfügung gestellt.

In den Figuren 3 und 4 sind drei weitere Aufnahmeöffnungen 86, 88, 90 am Grundgehäuseteil 24 zu erkennen, die sich parallel zu den Kanälen 70, 72, 74, 76 erstrecken. In der mittleren in der Symmetrieebene 56 angeordneten Aufnahmeöffnung befindet sich ein Temperaturfühler 92, der beispielsweise als PTC-Element ausgebildet ist und sich zwischen den beiden anderen Aufnahmeöffnungen 88, 90 befindet, in denen jeweils eine Heizpatrone 94 angeordnet ist, welche dafür sorgen, dass im Konverter 26 eine Temperatur von etwa 280°C herrscht, welches die Temperatur ist, bei der eine optimale Reaktion verläuft. Auch diese Kanäle werden durch die Platte 82 verschlossen.

Neben der bereits beschriebenen guten Zugänglichkeit zum Einbau und Ausbau des Konverters 26 ist dieser auch so ausgeführt, dass er bei zu kleinem vertikal zur Verfügung stehendem Bauraum waagerecht eingebaut werden kann, indem er im Vergleich zur dargestellten Einbaulage um seine Symmetrieachse um 180° gedreht und um in Richtung der Frontalebene nach vorne um 90° gekippt eingebaut wird. Die Kanäle sind für diesen Fall so angeordnet, dass lediglich die Durchströmungsrichtung des Grundgehäuseteils getauscht wird, im Übrigen jedoch keine konstruktiven Änderungen am Anschlussgehäuseteil oder am Grundgehäuseteil vorgenommen werden müssen. Dies führt zu einer vergrößerten Anzahl herzustellender Gleichteile, wodurch wiederum Kosten reduziert werden können.

## Patentansprüche

1. Analyseeinheit mit einer Frontalebene (28), von der aus die Analyseeinheit zugänglich ist und parallelen Befestigungsebenen (38), welche senkrecht zur Frontalebene (28) angeordnet sind, und
einem Gasmessgerät (12),
**dadurch gekennzeichnet, dass**
das Gasmessgerät (12) zumindest ein Anschlussgehäuseteil (22) aufweist, durch welches sich ein Einlasskanal (34) von einer der Befestigungsebenen (38) zu einer Anschlussebene (44) und ein Auslasskanal (36) von der Anschlussebene (44) bis zu der einen Befestigungsebene (38) erstreckt,
und ein Grundgehäuseteil (24) mit einer Anschlussebene aufweist, die der Anschlussebene (44) des Anschlussgehäuseteils (22) entspricht und an der das Grundgehäuseteil (24) gegen das Anschlussgehäuseteil (22) anliegt, wobei im Grundgehäuseteil (24) ein Zuströmkanal (70) ausgebildet ist, der fluidisch über die Anschlussebene (44) mit dem Einlasskanal (34) des Anschlussgehäuseteils (22) verbunden ist und ein Rückströmkanal (76) ausgebildet ist, der fluidisch über die Anschlussebene (44) mit dem Auslasskanal (36) des Anschlussgehäuseteils (22) verbunden ist,
wobei die korrespondierenden Anschlussebenen (44) im 45°-Winkel von der einen Befestigungsebene (38) in Richtung zur Frontalebene (28) gekippt angeordnet sind.

2. Analyseeinheit nach Anspruch 1,
**dadurch gekennzeichnet, dass**
alle Bohrungen (48, 66), Kanäle (34, 36, 70, 72, 74, 76), Aufnahmeöffnungen (86, 88, 90) oder Ausnehmungen (50, 52, 54) im Anschlussgehäuseteil (22) und im Grundgehäuseteil (24) symmetrisch zu einer zentralen Symmetrieebene (56) angeordnet sind, die senkrecht zu der einen Befestigungsebene (38) verläuft.

3. Analyseeinheit nach einem der Ansprüche 1 oder 2,
**dadurch gekennzeichnet, dass**
das Grundgehäuseteil (24) eine Flanschplatte (58) aufweist, deren erste Flanschfläche (60) die Anschlussebene (44) bildet und deren zweite Flanschfläche (62) parallel zur Anschlussebene (44) verläuft, wobei sich Bohrungen (66) von der ersten Flanschfläche (60) zur zweiten Flanschfläche (62) durch die Flanschplatte (58) erstrecken.

4. Analyseeinheit nach Anspruch 3,
**dadurch gekennzeichnet, dass**
am Anschlussgehäuseteil (22) zwei Gewindebohrungen (48) ausgebildet sind, in welche Schrauben (68) drehbar sind, die sich durch die Bohrungen (66) der Flanschplatte (58) in die Gewindebohrungen (48) erstrecken.

5. Analyseeinheit nach Anspruch 2 und 4, **dadurch gekennzeichnet, dass** sich ein Körper (64) des Grundgehäuseteils (24) zwischen den beiden Bohrungen (66) im 45° Winkel zu den Flanschflächen (60, 62) erstreckt, wobei das Grundgehäuseteil (24) in zwei senkrecht zueinander angeordneten Positionen am Anschlussgehäuseteil (22) befestigbar ist.

6. Analyseeinheit nach einem der vorhergehenden Ansprüche,
**dadurch gekennzeichnet, dass**
die Anschlussebene (44) des Anschlussgehäuseteils (22) in eine Auflagefläche (46) des Anschlussgehäuseteils übergeht, die parallel zu der einen Befestigungsebene (38) angeordnet ist.

7. Analyseeinheit nach Anspruch 6,
**dadurch gekennzeichnet, dass**
die Anschlussebene (44) des Grundgehäuseteils (24) in eine Fläche (62) übergeht die um 45° zur Anschlussebene (44) abgewinkelt ist und die auf die Auflagefläche (46) des Anschlussgehäuseteils (22) in einer ersten Befestigungsposition aufsetzbar ist.

8. Analyseeinheit nach einem der vorhergehenden Ansprüche,
**dadurch gekennzeichnet, dass**
die Analyseeinheit ein Konvertermaterial, welches zur Umwandlung von Kohlenwasserstoffen in Methan geeignet ist, aufweist, wobei das Konvertermaterial im Zuströmkanal (70) und/oder im Rückströmkanal (76) des Grundgehäuseteils angeordnet ist.

9. Analyseeinheit nach Anspruch 8,
**dadurch gekennzeichnet, dass**
im Grundgehäuseteil (24) zwischen dem Zuströmkanal (70) und dem Rückströmkanal (76) zwei weitere parallel verlaufende Durchströmungskanäle (72, 74) ausgebildet sind, in denen das Konvertermaterial angeordnet ist und die mit dem jeweils benachbarten Zuströmkanal (70) beziehungsweise Rückströmkanal (76) über axiale Ausnehmungen (78) zwischen den vom Anschlussgehäuseteil (22) weg weisenden Enden des Zuströmkanals (70) und des benachbarten Durchströmungskanals (72) und des Rückströmkanals (76) und des benachbarten Durchströmungskanals (74) miteinander verbunden sind.

10. Analyseeinheit nach Anspruch 9,
**dadurch gekennzeichnet, dass**
im Grundgehäuseteil (24) am zum Anschlussgehäuseteil (22) weisenden Ende ein Verbindungskanal (80) zwischen den beiden Durchströmungskanälen (72, 74) ausgebildet ist, so dass das Grundgehäuseteil (24) mäanderförmig durchströmbar ist.

11. Analyseeinheit nach einem der Ansprüche 9 oder 10,
**dadurch gekennzeichnet, dass**
an der Anschlussebene (44) des Anschlussgehäuseteils (22) eine erste kreisförmige Ausnehmung (50) ausgebildet ist, in die der Einlasskanal (34) mündet und welche mit dem Zuströmkanal (70) des Grundgehäuseteils (24) fluidisch verbunden ist und eine zweite kreisförmige Ausnehmung (52) ausgebildet ist, in die der Rückströmkanal (76) des Grundgehäuspteils (24) mündet und welche mit dem Auslasskanal (36) des Anschlussgehäuseteils (22) fluidisch verbunden ist, und eine langlochförmige Ausnehmung (54) ausgebildet ist, in die die beiden Durchströmungskanäle (72, 74) des Grundgehäuseteils (24) münden.

12. Analyseeinheit nach einem der Ansprüche 9 bis 11,
**dadurch gekennzeichnet, dass**
der Zuströmkanal (70), der Rückströmkanal (76) und die beiden Durchströmungskanäle (72, 74) mittels einer an ihrem zum Anschlussgehäuseteil (22) entgegengesetzten Ende aufgeschraubten Platte (82) verschlossen sind.

13. Analyseeinheit nach einem der vorhergehenden Ansprüche,
**dadurch gekennzeichnet, dass**
die Analyseeinheit eine Heizpatrone (54) und einen Temperaturfühler (92) aufweist, wobei im Grundgehäuseteil (24) zumindest eine erste Aufnahmeöffnung (88; 90) ausgebildet ist, in der die Heizpatrone (94) angeordnet ist und zumindest eine zweite Aufnahmeöffnung (86) ausgebildet ist, in der der Temperaturfühler (92) angeordnet ist.

14. Analyseeinheit nach einem der vorhergehenden Ansprüche,
**dadurch gekennzeichnet, dass**
das Gasmessgerät (12) ein Flammenionisationsdetektor ist und das Grundgehäuseteil (24) einen Konverter (26) bildet.

## Claims

1. Analysis unit comprising
a frontal plane (28), from which the analysis unit is accessible, and parallel mounting planes (38), which are arranged perpendicularly to the frontal plane (28); and
a gas measurement device (12),
**characterized in that**
the gas measurement device (12) has at least one connection housing part (22), through which an inlet channel (34) extends from the mounting plane (38) to a connection plane (44) and through which an outlet channel (36) extends from the connection plane (44) to the one mounting plane (38); and
a main housing part (24) with a connection plane that corresponds to the connection plane (44) of the connection housing part (22) and on which the main housing part (24) rests against the connection housing part (22), wherein the main housing part (24) is equipped with an inflow channel (70), which is in fluid communication with the inlet channel (34) of the connection housing part (22) via the connection plane (44), and a recirculation channel (76), which is in fluid communication with the outlet channel (36) of the connection housing part (22) via the connection plane (44),
wherein the corresponding connection planes (44) are tilted at an angle of 45° from the mounting plane (38) in direction of the frontal plane (28).

2. Analysis unit according to claim 1,
**characterized in that**
all bores (48, 66), channels (34, 36, 70, 72, 74, 76), receiving openings (86, 88, 90) or recesses (50, 52, 54) in the connection housing part (22) and in the main housing part (24) are arranged symmetrically relative to a symmetry plane (56) running perpendicular to the mounting plane (38).

3. Analysis unit according to one of the claims 1 or 2,
**characterized in that**
the main housing part (24) comprises a flange plate (58), whose first flange face (60) forms the connection plane (44) and whose second flange face (62) runs parallel to the connection plane (44), wherein bores (66) extend from the first flange face (60) to the second flange face (62) through the flange plate (58).

4. Analysis unit according to claim 3,
**characterized in that**
the connection housing part (22) is equipped with two threaded holes (48), wherein screws (68) can be tightened into the threaded holes, which extend through the bores (66) of the flange plate (58) into the threaded holes (48).

5. Analysis unit according to claims 2 and 4,
**characterized in that**
a body (64) of the main housing part (24) extends between the two bores (66) at an angle of 45° relative to the flange faces (60, 62), wherein the main housing part (24) can be mounted in two perpendicularly arranged positions on the connection housing part (22).

6. Analysis unit according to any one of the preceding claims,
**characterized in that**
the connection plane (44) of the connection housing part (22) merges into a contact face (46) of the connection housing part (22), which is arranged parallel to the one mounting plane (38).

7. Analysis unit according to claim 6,
**characterized in that**
the connection plane (44) of the main housing part (24) merges into a face (62), which is angled by 45° relative to the connection plane (44) and can be placed on the contact face (46) of the connection housing part (22) in a first mounting position.

8. Analysis unit according to any one of the preceding claims,
**characterized in that**
the analysis unit includes a converter material which is used to convert hydrocarbon into methane, said converter material being arranged in the inflow channel (70) and/or the recirculation channel (76) of the main housing part.

9. Analysis unit according to claim 8,
**characterized in that**
in the main housing part (24) between the inflow channel (70) and the recirculation channel (76) two further parallel flow channels (72, 74) are formed in which the converter material is arranged and which are connected to each other with the respective adjacent inflow channel (70) or recirculation channel (76) via axial recesses (78) between the ending of the inflow channel (70) facing away from the connection housing part (22) and the adjacent flow channel (72) and the recirculation channel (76) and the adjacent flow channel (74).

10. Analysis unit according to claim 9,
**characterized in that**
in the main housing part (24) at the ending facing to the connection housing part (22) between the two flow channels (72, 74) a connection channel (80) is formed so that the main housing part (24) is flowed in a meandering way.

11. Analysis unit according to one of the claims 9 or 10,
**characterized in that**
the connection plane (44) of the connection housing part (22) is equipped with a first circular recess (50) into which the inlet channel (34) leads and which is in fluid communication with the inflow channel (70) of the main housing part (24), and with a second circular recess (52) into which the at least one recirculation channel (76) of the main housing part (24) leads and which is in fluid communication with the outlet channel (36) of the connection housing part (24), and with an oblong recess (54) into which the two flow channels (72, 74) of the main housing part (24) lead.

12. Analysis unit according to one of the claims 9 to 11,
**characterized in that**
the inflow channel (70), the recirculation channel (76) and the two flow channels (72, 74) are closed with a plate (82) screwed on their respective endings opposite to the connection housing part (22).

13. Analysis unit according to any one of the preceding claims,
**characterized in that**
the analysis unit comprises a heating cartridge (54) and a temperature sensor (92), the main housing part (24) being equipped with at least one first receiving opening (88, 90) in which the heating cartridge (94) is arranged and in which at least one second receiving opening (86) is formed in which the temperature sensor (92) is arranged.

14. Analysis unit according to one of the preceding claims,
**characterized in that**
the gas measurement device (12) is a flame ionization detector and the main housing part (24) forms a converter (26).

## Revendications

1. Unité d'analyse comprenant un plan frontal (28) à partir duquel l'unité d'analyse est accessible et des plans de fixation parallèles (38) qui sont disposés perpendiculairement par rapport au plan frontal (28),
un dispositif de mesure de gaz (12),
**caractérisée en ce que**
un dispositif de mesure de gaz (12) présente au moins une partie de carter de connexion (22) à travers laquelle un canal d'entrée (34) s'étend à partir du plan de fixation (38) jusqu'à un plan de connexion (44) et un canal de sortie (36) s'étend à partir du plan de connexion (44) jusqu'au plan de fixation (38),
et comporte une partie de carter de base (24) présentant un plan de connexion qui correspond au plan de connexion (44) de la partie de carter de connexion (22) et auquel la partie de carter de base (24) repose contre la partie de carter de connexion (22), un canal d'afflux (70) étant formé dans la partie de carter de base (24) qui est connecté fluidiquement via le plan de connexion (44) au canal d'entrée (34) de la partie de carter de connexion (22) et un canal de retour (76) est formé qui est connecté fluidiquement au canal de sortie (36) de la partie de carter de connexion (22) via le plan de connexion (44),
les plans de connexion correspondants (44) étant disposés inclinés sous un angle de 45° par rapport au plan de fixation (38) vers le plan frontal (28).

2. Unité d'analyse selon la revendication 1, **caractérisée en ce que** tous les alésages (48, 66), canaux (34, 36, 70, 72, 74, 76), ouvertures de réception (86, 88, 90) ou évidements (50, 52, 54) dans la partie carter de raccordement (22) et dans la partie carter de base (24) sont disposées symétriquement par rapport à un plan central de symétrie (56) perpendiculaire par rapport au plan de fixation (38).

3. Unité d'analyse selon l'une des revendications 1 ou 2, **caractérisée en ce que** la partie de carter de base (24) comprend une plaque de bride (58), dont la première surface de bride (60) forme le plan de connexion (44) et la deuxième surface de bride (62) est parallèle par rapport au plan de connexion (44), des alésages (66) s'étendant à partir de la première surface de bride (60) jusqu'à la deuxième surface de bride (62) à travers la plaque de bride (58).

4. Unité d'analyse selon la revendication 3, **caractérisée en ce que** deux alésages filetés (48) sont formés dans la partie de carter de raccordement (22), dans lesquels des vis (68) peuvent être tournées, qui s'étendent à travers les alésages (66) de la plaque de bride (58) dans les alésages filetés (48).

5. Unité d'analyse selon les revendications 2 et 4, **caractérisée en ce qu'**un corps (64) de la partie de carter de base (24) s'étend entre les deux alésages (66) sous un angle de 45° par rapport aux surfaces de bride (60, 62), la partie de carter de base (24) pouvant être fixée à la partie de carter de connexion (22) dans deux positions mutuellement perpendiculaires.

6. Unité d'analyse selon l'une quelconque des revendications précédentes, **caractérisée en ce que** le plan de connexion (44) de la partie de carter de connexion (22) passe dans une surface de support (46) qui est disposée parallèlement par rapport ledit plan de fixation (38).

7. Unité d'analyse selon la revendication 6, **caractérisée en ce que** le plan de connexion (44) de la partie de carter de base (24) passe dans une surface (62) qui est inclinée à 45° par rapport au plan de connexion (44) et qui peut être placée sur la surface de support (46) de la partie de carter de connexion (22) dans une première position de fixation.

8. Unité d'analyse selon l'une quelconque des revendications précédentes, **caractérisée en ce qu'**un matériau de convertisseur, qui est utilisé pour convertir les hydrocarbures en méthane, est disposé dans le canal d'entrée (70) et/ou dans le canal de retour (76) de la partie de carter de base (24)

9. Unité d'analyse selon la revendication 8, **caractérisée en ce que** dans la partie de carter de base (24) entre le canal d'entrée (70) et le canal de retour (76), deux autres canaux d'écoulement parallèles (72, 74) sont formés, dans lesquels ledit matériau de convertisseur est disposé et qui sont raccordés au canal d'entrée (70) ou de retour (76) respectivement adjacent par des évidements axiaux (78) entre les extrémités du canal d'entrée (70) opposées à la partie de carter de connexion (22) et le canal d'écoulement adjacent (72) et le canal de retour (76) et le canal d'écoulement adjacent (74).

10. Unité d'analyse selon la revendication 9, **caractérisée en ce que** dans la partie de carter de base (24), à l'extrémité tournée vers la partie de carter de connexion (22), un canal de connexion (80) est formé entre les deux canaux d'écoulement (72, 74) de sorte que la partie de carter de base (24) peut être traversé de manière sinueuse.

11. Unité d'analyse selon l'une des revendications 9 ou 10, **caractérisée en ce qu'**un premier évidement circulaire (50) est formé dans le plan de connexion (44) de la partie de carter de connexion (22), dans lequel s'ouvre le canal d'entrée (34) et qui est raccordé fluidiquement au canal d'entrée (70) de la partie de carter de base (24), et un deuxième évidement circulaire (52), dans lequel ledit un canal de retour (76) de la partie de carter de base (24) s'ouvre et qui est raccordé fluidiquement au canal de sortie (36) de la partie de carter de connexion (22), et un évidement en forme de trou élongé (54) est formé dans lequel s'ouvrent les deux canaux d'écoulement (72, 74) de la partie de carter de base (24).

12. Unité d'analyse selon l'une des revendications 9 à 11, **caractérisée en ce que** le canal d'entrée (70), le canal de retour (76) et les deux canaux de passage (72, 74) sont fermés au moyen d'une plaque (82) vissée sur leur extrémité opposée à la partie de carter de raccordement (22).

13. Unité d'analyse selon l'une quelconque des revendications précédentes, **caractérisée en ce que** l'unité d'analyse comprend une cartouche de chauffage (54) et un capteur de température (92), au moins une première ouverture de réception (88; 90) étant formée dans la partie de carter de base (24), dans laquelle ouverture la cartouche de chauffage (94) est disposée, et au moins une deuxième ouverture de réception (86) étant formée, dans laquelle le capteur de température (92) est disposé.

14. Unité d'analyse selon l'une quelconque des revendications précédentes, **caractérisée en ce que** le dispositif de mesure de gaz (12) est un détecteur à ionisation de flamme et la partie de carter de base (24) forme un convertisseur (26).
